(19) Europäisches Patentamt
European Patent Office
Office européen des brevets

(11) Publication number: **0 254 223 A1**

# (12) EUROPEAN PATENT APPLICATION

(21) Application number: 87110364.4

(22) Date of filing: 17.07.87

(51) Int. Cl.4: C07D 257/02 , A61K 31/395 , C12P 17/10

(30) Priority: 22.07.86 JP 170856/86

(43) Date of publication of application: 27.01.88 Bulletin 88/04

(84) Designated Contracting States: AT BE CH DE ES FR GB GR IT LI LU NL SE

(71) Applicant: MICROBIAL CHEMISTRY RESEARCH FOUNDATION
14-23, Kamiosaki 3 Chome Shinagawa-ku
Tokyo 141(JP)

(72) Inventor: Umezawa, Hamao

Deceased(DE)
Inventor: Okami, Yoshiro
4-18-14, Denenchofu
Ota-ku Tokyo(JP)
Inventor: Kurasawa, Shogo
5-39-2, Sendagi
Bunkyo-ku Tokyo(JP)
Inventor: Kameyama, Toshiuki
5-10-26, Nishikasai
Edogawa-ku Tokyo(JP)
Inventor: Takahashi, Atsushi
4-25-18, Nishiikuta
Kawasaki City Kanagawa Prefecture(JP)
Inventor: Ishizuka, Masaaki
3-17, Denenchofu-honcho
Ota-ku Tokyo(JP)

(74) Representative: Becker, Heinrich Karl Engelbert, Dr. et al
HOECHST AKTIENGESELLSCHAFT Central Patent Department P.O. Box 80 03 20
D-6230 Frankfurt am Main 80(DE)

## (54) Bisucaberin, a process for producing this compound and its use as a medicament.

(57) This invention relates to bisucaberin (1,12-dihydroxy-1,6,12,17-tetraazacyclodocosan-2,5,13,16-tetrone) of the following formula I



Xerox Copy Centre

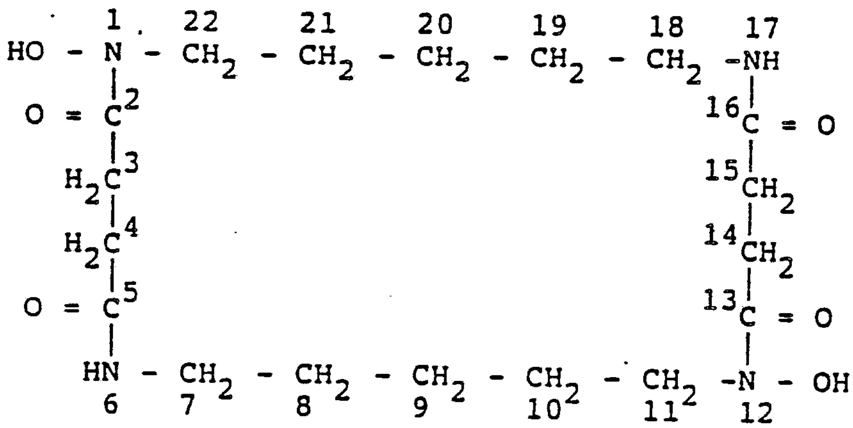

and a process for producing this compound.

## Bisucaberin, a process for producing this compound and its use as a medicament

This invention relates to bisucaberin (1,12-dihydroxy1,6,12,17-tetraazacyclodocosan-2,5,13,16-tetrone) of the following formula I

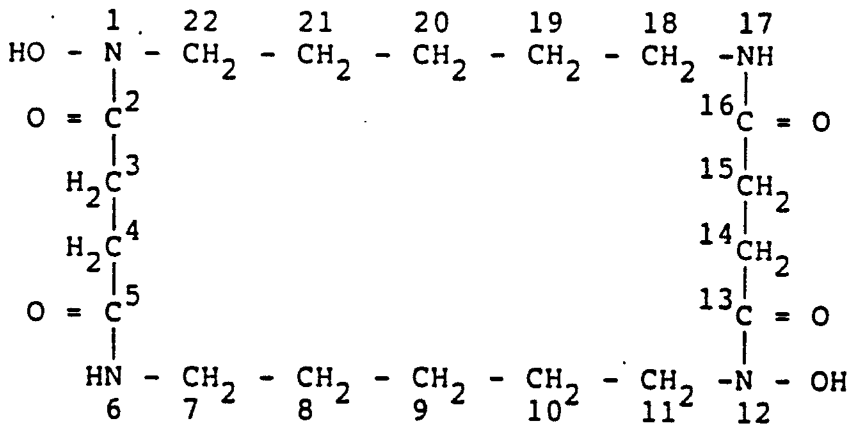

and a process for producing this compound.

Numerous antibiotics produced by microorganisms are known, and many reports have issued on substances having antitumor activity.

Now it has been found that a microorganism of the genus Anteromonas produces a novel substance having antitumor activity.

Consequently, this invention provides bisucaberin of the following formula I

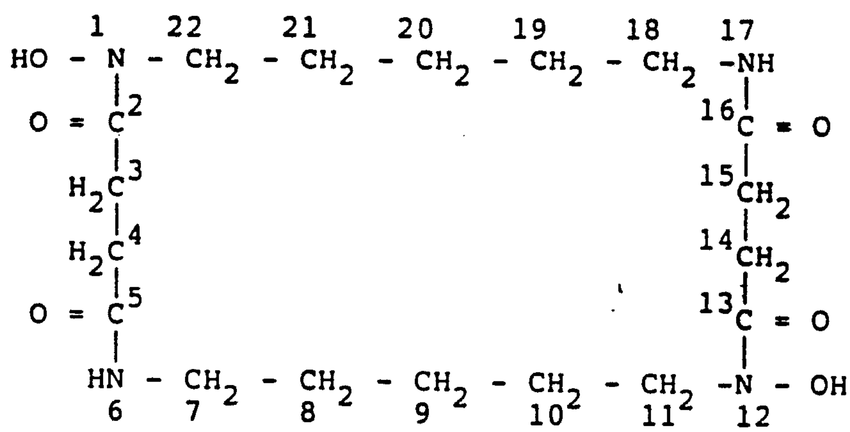

and a process for producing bisucaberin which comprises culturing a microorganism belonging to the genus Alteromonas and having the ability to produce bisucaberin, and then recovering bisucaberin from the cultured broth.

The chemical name for bisucaberin is 1,12-dihydroxy-1,6,12,17-tetraazacyclodocosan-2,5,13,16-tetrone. This substance has the following physicochemical properties:

(a) Appearance: Colorless crystals

(b) Melting point: 190°C (decomposition)

(c) Ultraviolet absorption spectrum:

$\lambda max(\epsilon)$ = 215 nm (5740) in methanol, 215 nm (5700) in 0.1 N hydrochloric acid-methanol

(d) Molecular weight:

m/Z = 401 ($MH^+$) by SIMS (secondary ion mass spectrometry)

m/Z = 401 ($MH^+$) by EIMS (electron ion mass spectrometry)

(e) Molecular formula: $C_{18}H_{32}N_4O_6$

(f) Elemental analysis:

| | C % | H % | N % | (O %) |
|---|---|---|---|---|
| Found: | 53.69 | 7.97 | 13.20 | (25.14) |
| Calculated: | 54.00 | 8.00 | 14.00 | (24.00) |

(g) Infrared absorption spectrum: Shown in Figur 1.

(h) Proton NMR spectrum: Shown in Figur 2.

(i) $^{13}C$-NMR spectrum: Shown in Figur 3.

(j) Solubility:

Soluble in dimethyl sulfoxide, slightly soluble in methanol, and sparingly soluble or insoluble in water.

(k) Color reactions:

Positive in molybdenum-sulfuric acid reaction, tolidine reaction and ferric chloride reaction, and negative in ninhydrin reaction

(l) Thin-layer chromatography on silica gel (art 5715, Merck):

$R_f$ = 0.52 for chloroform:methanol (90:10)

$R_f$ = 0.38 for chloroform:methanol (92:8)

(m) High-voltage paper electrophoresis (3000 V, 20 min):

Rm = 0.0 for formic acid:acetic acid:water (1:3:36) given as relative mobility with the mobility of alanine taken as 1.0.

None of the known substances have the above-mentioned characteristics. Accordingly, bisucaberin was judged to be a novel substance.

Bisucaberin in accordance with this invention is present in the cultured broth of bisucarberin-producing microorganism of the genus Alteromonas, and can be recovered therefrom. An example of the bisucaberin-producing microorganism is the strain Alteromonas haloplanktis SB-1123. This strain was isolated by us from a soil sample that had been collected at the bottom of a deep-sea region off Aomori Prefecture, Japan. It has the following microbiological characteristics:

(a) Morphological characteristics

1) Shape and dimensions of cells: Rod, 0.5-0.8 µm x 1.5-2.0 µm
2) Mobility: Motile
3) Spores: None
4) Grams's stain: Negative

(b) Cultural characteristics on various media

1) On buillon-agar plate culture medium: No growth
2) On Z medium-agar plate culture medium: Moderate growth, circular, flat to convex, entire, smooth, wet-luminescent, translucent, light yellow (Z medium: Sea water at pH 7.2 containing 0.5 % polypeptone and 0.1 % yeast extract)
3) On buillon-agar slant culture medium: No growth
4) On Z medium-agar slant culture medium: Moderate growth, somewhat spreading, wet-luminescent, buttery light yellow
5) On buillon-liquid culture medium: No growth
6) On buillon-sea water liquid culture medium: Intermediate growth
7) On buillon-gelatine stab culture medium: No changes
8) On buillon-sea water-gelatin stab sulture medium: Liquefaction

(c) Physiological characteristics

1) Reduction of nitrates: Negative
2) Denitrification reaction: Negative
3) MR test: Negative
4) VP test: Negative

5) Production of hydrogen sulfide: Negative

6) Hydrolysis of starch: Negative

7) Production of pigments: No production of water-soluble pigments

8) Oxidase: Positive

9) Catalase: Positive

10) Growth ranges:

Temperature Good growth at 10 to 34°C (optimal range: 14 to 30°C), no growth at 4°C and 39°C.
pH 6 to 9

11) Behavior toward oxygen: Aerobic

12) O-F test by the Hugh & Leifson method: No production of acids

13) Decarboxylase reaction: Negative for lysine and negative for arginine

14) Nutrient requirement: No growth in the absence of sea water or an inorganic salts solution of a similar composition

15) Utilization of compounds:

D-Glucose + ; N-Acetyl glucosamine + ;
D-Mannose + ; Succinic acid + ;
D-Fructose + ; Fumaric acid + ;
Saccharose + ; Citric acid + ;
Maltose + ; Aconitic acid - ;
Cellobiose - ; Erithritol - ;
Melibiose - ; Mannitol - ;
Lactose - ; Glycerol - ;
L-Threonine - ; $\gamma$-Aminobutyric acid - ;
L-Thyrosine ± ; D-Sorbitol - ;
Putrescine - ; Maleic acid - ;
Starch ± ; $\alpha$-Ketoglutaric acid - ;
Gluconic acid -.

16) GC content of DNA: 44.1 %

The above-mentioned microbiological characteristics of the microorganism are summarized as follows:

(1) The microorganism is a marine bacterium.

(2) It grows only under aerobic conditions.

(3) It is a Gram-negative bacillus.

(4) It is motile with polar flagella.

(5) It decomposes saccharides oxidatively and utilizes saccharides.

(6) It has a DNA-GC content of 44.1 %

Searching made on Bergey's Manual of Systematic Bacteriology, Vol. 1 (1984) in the light of the above characteristics has shown that the above microorganism closely relates to the genus Alteromonas. Hence, the microorganism was identified as a bacterium of the genus Alteromonas.

Subsequent search on species has shown the microorganims to agree with Alteromonas haloplanktis based on the following important characteristics: it does not grow at 4°C; it can utilize such compounds as D-mannose, saccharose, maltose, N-acetyl glucosamine, succinic acid, fumaric acid and citric acid, as carbon sources; it cannot utilize compounds, such as erithritol, glycerol, sorbitol, maleic acid and $\alpha$-keto glutaric acid, as carbon sources; and it does not produce soluble pigments. Although its inability to utilize aconitic acid as a carbon source is inconsistent with the description in the literature, it is not a significant difference as to distinguish the microorganism from the species Alteromonas haloplanktis. Accordingly, the microorganism was identified to be Alteromonas haloplanktis (ZoBell and Upham 1944) Reichelt and Baumann 1973.

The microorganism was deposited as Alteromonas haloplanktis SB-1123 with the Fermentation Research Institute, Agency of Industrial Science and Technology, Ministry of International Trade and Industry of Japan, where it was assigned FERM P-8803. In addition to this microorganism, this invention can use any of its variants obtained by its artificial or spontaneous mutation, as long as they have the ability to produce bisucaberin.

The culture of the bisucaberin-producing microorganism is carried out using a culture medium prepared by dissolving carbon sources, nitrogen sources, inorganic ions, and if desired, vitamins and amino acids, in sea water (including artificial sea water). The carbon sources may be those in customary use for the culture of microorganisms. Preferred examples are carbohydrates such as glucose, sucrose, starch and dextrin. The nitrogen sources may be those which are in customary use for the cultivation of microorganisms.

Examples include peptone, yeast extract, meat extract, corn steep liquor, soybean meal, casein, and ammonium ions. For the efficient production of bisucaberin, the recommendable method is to use a culture medium of a processed form, say in ground form, of dried cuttlefish and/or dried sardine, preferably that of Eugraulis japonica, in artificial sea water.

The culture should preferably be liquid cultur under agitation and aeration. The culture temperature is usually 10 to 35°C, preferably 24 to 30°C. The culture should be continued until a sufficient amount of bisucaberin is accumulated in the cultured broth. Usually, this is attained in 16 hours to 5 days.

The recovery of bisucaberin from the cultured broth can be done by a known method. An example of it comprises centrifuging the culture broth to obtain a supernatant, passing it through a layer of a synthetic absorbent resin to give fractions followed by concentrating them to precipitate crystals, and purifying them.

Bisucaberin of this invention has antitumor activity. For example, it enhances the cytolytic activity of macrophages against cancer cells and inhibits the proliferation of cancer cells.

This invention will be described in more detail with reference to the Examples but is not limited to them.

## Example 1

The strain Alteromonas haloplanktis SB-1123 (FERM P-8803) was inoculated into 10 liters of a culture medium (pH 7,2) containing 2 % of cuttlefish and 1 % of dried sardine (Eugraulis japonica), both in ground form, in arficial sea water (Jamarin S, a product of Jamarin Laboratory Inc). diluted to a half concentration. The inoculum was cultured under shake for 3 days at 27°C.

The cultured medium collected was centrifuged to obtain a supernatant. 7 liters of the supernatant was passed through a column of Diaion HP-20 (500 ml), which was washed with 5 liters of water. Then, the column was eluted with a 50:50 mixture of acetone and water, and 2 liters of active fractions was collected. The combined active fraction was concentrated under reduced pressure to precipitate crude crystals. The crystals were washed with acetone and then with ethyl acetate to give crystals of bisucaberin in an amount of 2 g.

If the crystals were brown due to the incorporation of impurities therein, they can be easily converted into colorless, plate-like, pure crystals of bisucaberin in the following manner: They are subjected to silica gel column chromatography using chloroform-methanol (96:4) as developing solvent. The active fractions are combined and concentrated to obtain pure bisucaberin.

## Example 2

The effect of bisucaberin on the cytolytic activity of macrophages against cancer cells was investigated.

Macrophages were obtained from peritoneal exudate cells induced by the intraperitoneal injection of 10 % proteosepeptone (Difco) to 8-weel old female C3H/HeN mice, followed by the removal of cells non-adherent to plastics. Target cancer cells were fibrosarcoma L-1023 cells induced in the C3H/HeN mice with methyl cholanthrene, said cells being preliminary labelled with tritiated thymidine. The culture of the cells was performed in an RPMI 1640 culture medium at 37°C in the presence of 5 % carbon dioxide.

A 96-well microplate was charged with 2 x $10^5$ cells/well of the macrophages and 1 x $10^4$ cells/well of the target cancer cells, and incubated for 2 days in the presence of a predetermined amount of bisucaberin. Then, the amount of tritium released from the lysed cancer cells into supernatant of the cultured broth was measured. The results are shown in Tabel 1. The cytolytic activity was represented by % cytolysis calculated from the equation:

Cytolysis (%) = [(amount of experimental release) - (amount of spontaneous release)]/[(amount of maximum release) - (amount of spontaneous release)],

in which (amount of spontaneous release) was the value when no sample was added to the culture and (amount of maximum release) was the value when 0.5 % SDS was added in place of the macrophages.

Table 1

| Amount of bisucaberin added (ug/ml, final concentration) | Cytolysis (%) + Macrophages (%) | | - Macrophages (%) |
|---|---|---|---|
| 67 | 38.6 | 41.5 | 15.3 |
| 33 | 64.8 | 71.8 | 12.0 |
| 11 | 45.7 | 47.1 | 6.5 |
| 3.6 | 13.3 | 14.8 | 0.3 |
| 1.2 | 2.8 | - | - |
| 0 | 0 | - | 0 |

Example 3

The activity of bisucaberin to inhibit the growth of mouse leukemia L-1210 cells and mouse IMC carcinoma cells was investigated . The experimental conditions were as follows:

(1) Tumor cells

a) L-1210: Cultured in passage in an MEM culture medium containing 10 % calf serum.

b) IMC carcinoma: Cultured in passage in an RPMI 1640 culture medium containing 10 % fetal bovine serum.

The tumor cells in the exponential phase were used in experiments with each culture started on the day before the experiments.

(2) Sample

Bisucaberin was dissolved in dimethyl sulfoxide whose volume was 10 % of the volume of the final solution. To the solution was added 5 µl of Tween 80, and the mixture was adjusted to a concentration of 2 mg/ml with an MEM culture medium. This stock solution was diluted serially in six stages with the same culture medium, each time at a dilution ratio of 4.

(3) Method

a) A 96-well microplate was charged with 10 µl/well of the sample solution, with each sample solution dispensed into 3 wells for each one of the test specimens.

b) The tumor cells were suspended in the respective media for passage culture at a rate of 1 x $10^5$ cells per ml, and the suspensions were added to the microplate in an amount of 0.2 ml/well.

c) After 48 hours of culture at 37°C in a carbon dioxide incubator, 0.1 ml of the cell suspension was taken and diluted 100-fold with ISOTON II or a physiological saline solution. The number of cells in the resulting dilution was counted using a Coulter Counter.

d) The percent inhibition of cell growth was calculated for each dilution of the sample solution in comparison with the control. The results were plotted on a graph to prepare a curve of bisucaberin concentrations vs. % inhibition. From this curve, $IC_{50}$, the concentration of bisucaberin corresponding to 50 % inhibition, was determined. The % inhibition and $IC_{50}$ data are shown in Tables 2 and 3, respectively.

Table 2

| Tumor cells | Concentration of sample (μg/ml) | | Cell count | % inhibitor |
|---|---|---|---|---|
| L-1210 | Control | | 2888 ± 106 | - |
| | Bisucaberin | 100.0 | 429 ± 47 | 85 |
| | | 25.0 | 425 ± 3 | 85 |
| | | 6.25 | 908 ± 11 | 69 |
| | | 1.56 | 2428 ± 100 | 16 |
| | | 0.39 | 2727 ± 151 | 6 |
| IMC carcinoma | Control | | 3910 ± 96 | - |
| | Bisucaberin | 100.0 | 557 ± 26 | 86 |
| | | 25.0 | 547 ± 16 | 86 |
| | | 6.25 | 1712 ± 24 | 56 |
| | | 1.56 | 3374 ± 44 | 14 |
| | | 0.39 | 3602 ± 2 | 8 |

Table 3

| Tumor cells | $IC_{50}$ (μg/ml) |
|---|---|
| L-1210 | 3.9 |
| IMC carcinoma | 5.1 |

Brief Description of the Drawings (Fig. 1, 2 and 3)

Fig. 1 is the infrared absorption spectrum of bisucaberin.

Fig. 2 is the proton NMR spectrum of bisucaberin measured in $d_6$-DMSO solution with TMS as the reference material.

Fig. 3 is the $^{13}$C-NMR spectrum of bisucaberin measured in $d_6$-DMSO solution with DMSO as the reference material.

## Claims

1. The compound bisucaberin of the formula I

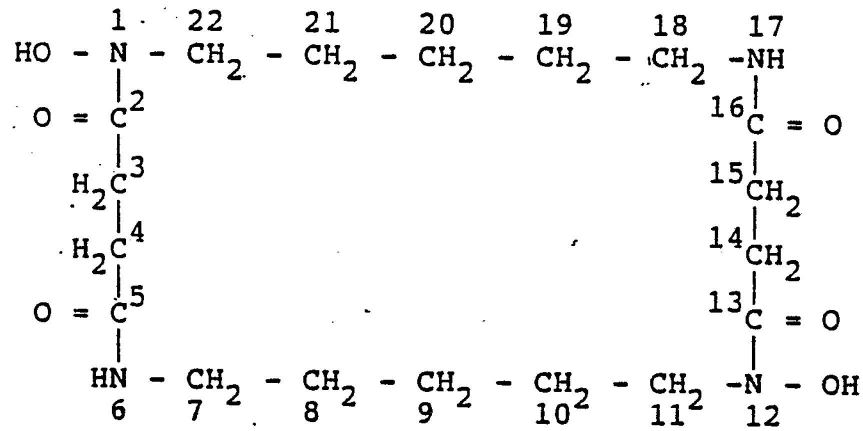

2. A process for producing bisucaberin of the formula I

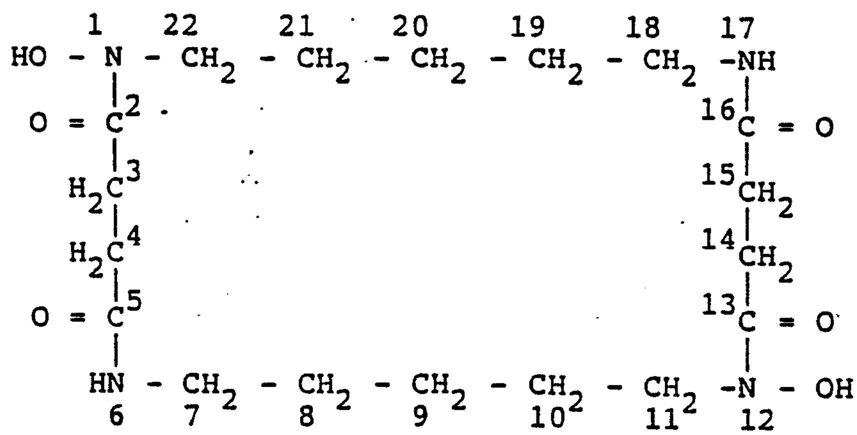

which comprises culturing a bisucaberin-producing microorganism of the genus Alteromonas, and recovering bisucaberin from the cultured broth.

3. The process of Claim 2 wherein the bisucaberin-producing microorganism of the genus Alteromonas is the strain Alteromonas haloplanktis SB-1123 (FERM P-8803).

4. A pharmaceutical composition comprising as the active ingredient the compound of the formula I as defined in claim 1 in association with a pharmaceutically acceptable carrier.

5. Use for the compound of the formula I as defined in claim 1 for the preparation of a medicament having antitumor activity.

Claims for the following contracting state: Austria

1. A process for producing bisucaberin of the formula I

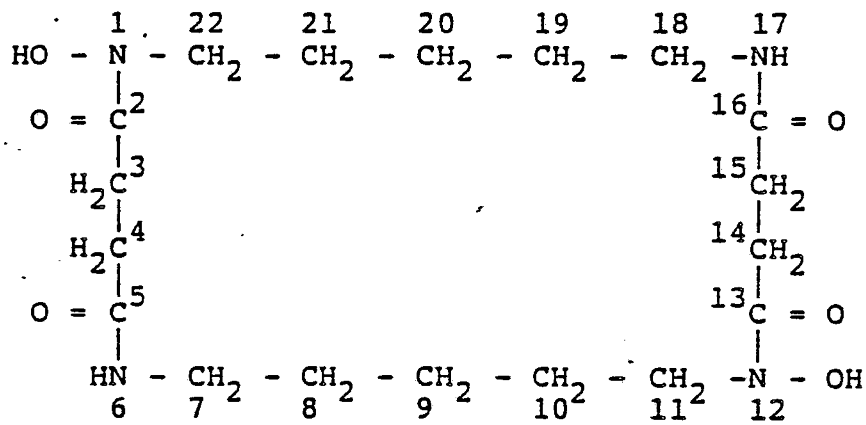

which comprises culturing a bisucaberin-producing microorganism of the genus Alteromonas, and recovering bisucaberin from the cultured broth.

2. The process of Claim 2 wherein the bisucaberin-producing microorganism of the genus Alteromonas is the strain Alteromonas haloplanktis SB-1123 (FERM P-8803).

FIG.1

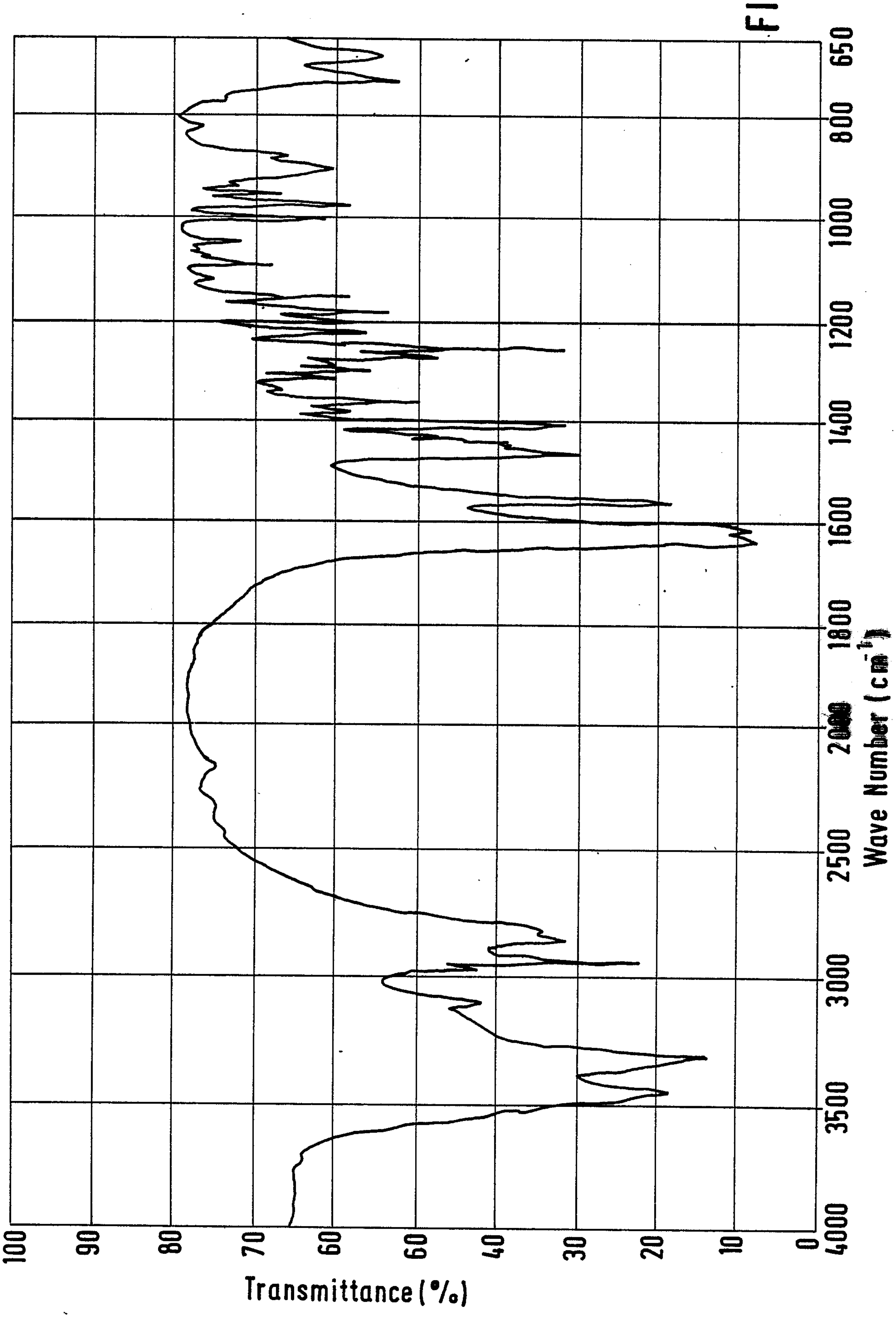
Transmittance (%)
100
90
80
70
60
50
40
30
20
10
0
Wave Number (cm⁻¹)
4000
3500
3000
2500
2000
1800
1600
1400
1200
1000
800
650

FIG. 2

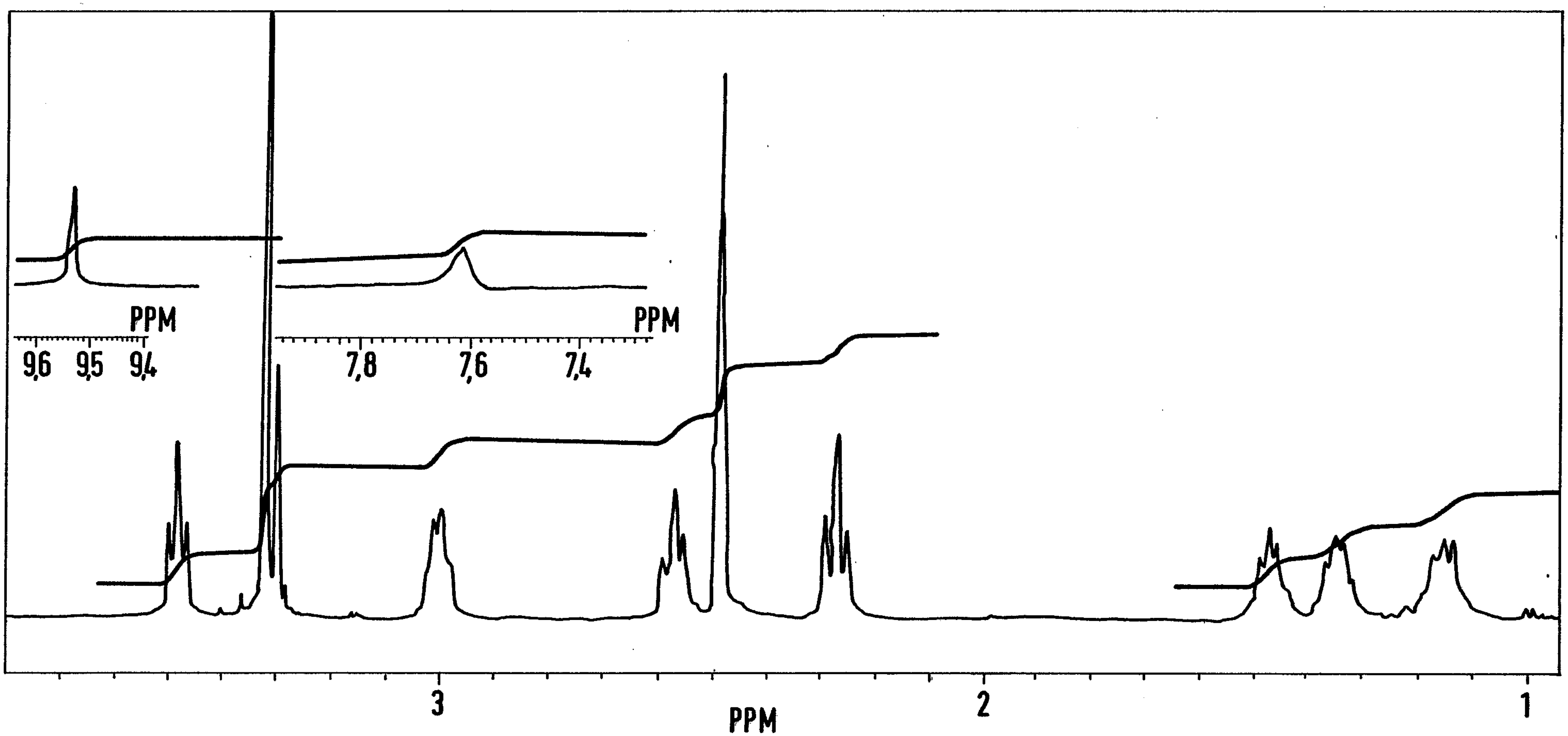

FIG.3

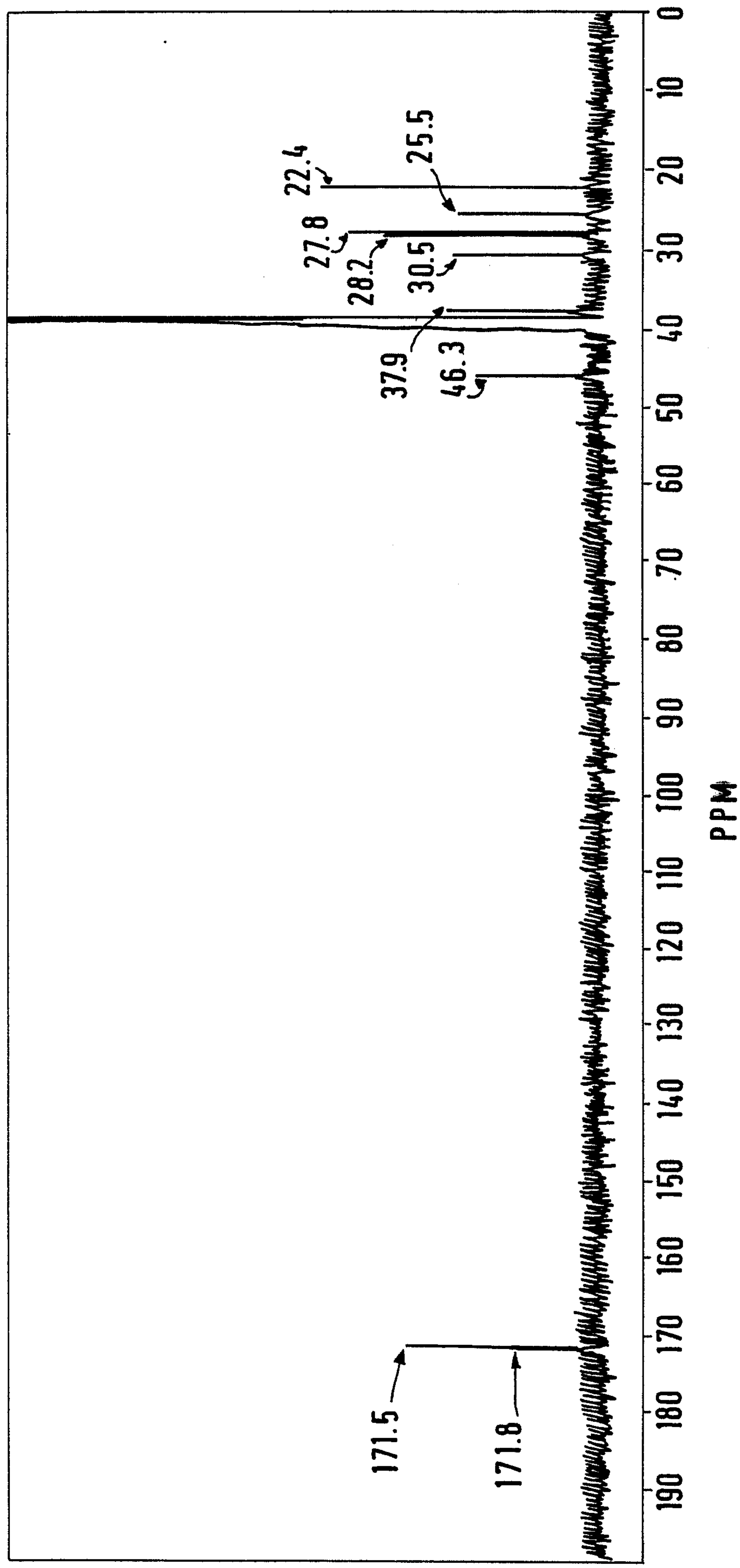
22.4
25.5
27.8
28.2
30.5
37.9
46.3
171.5
171.8
0
10
20
30
40
50
60
70
80
90
100
110
120
130
140
150
160
170
180
190
PPM

European Patent Office

# EUROPEAN SEARCH REPORT

Application number

EP 87110364.4

| DOCUMENTS CONSIDERED TO BE RELEVANT | | | |
|---|---|---|---|
| Category | Citation of document with indication, where appropriate, of relevant passages | Relevant to claim | CLASSIFICATION OF THE APPLICATION (Int. Cl.4) |
| A | CHEMICAL ABSTRACTS, vol. 98, no. 22, May 30, 1983, Columbus, Ohio, USA<br>ADAMS, HARRY; BAILEY, NEIL A.; FENTON, DAVID E.; LEAL GONZALEZ, MARTHA S.; PHILLIPS, CARL A. "Compartmental ligands. Part 4. The crystal and molecular structures of some oxovanadium(IV) complexes of acyclic ligands derived from heptane-2,4,6-trione and α,ω-alkanediamines"<br>page 695, column 2, abstract-no. 190 552a<br>& J. Chem. Soc., Dalton Trans. 1983, (2), 371-9<br>-- | 1 | C 07 D 257/02<br>A 61 K 31/395<br>C 12 P 17/10 |
| A | CHEMICAL ABSTRACTS, vol. 89, no. 5, July 31, 1978, Columbus, Ohio, USA<br>FENTON, DAVID E.; GAYDA, STEPHEN E. "Compartmental ligands. Part I. Mononuclear macrocyclic Schiff-base transition-metal complexes derived from 1,3,5-triketones"<br>page 630, column 1, abstract-no. 43 365c<br>& J. Chem. Soc., Dalton Trans. 1977, (21), 2095-101<br>-- | 1 | TECHNICAL FIELDS SEARCHED (Int. Cl.4)<br>C 07 D 257/00<br>C 12 P 17/00 |

The present search report has been drawn up for all claims

| Place of search | Date of completion of the search | Examiner |
|---|---|---|
| VIENNA | 10-09-1987 | HAMMER |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

European Patent Office

# EUROPEAN SEARCH REPORT

Application number



EP 87110364.4

## DOCUMENTS CONSIDERED TO BE RELEVANT

| Category | Citation of document with indication, where appropriate, of relevant passages | Relevant to claim | CLASSIFICATION OF THE APPLICATION (Int. Cl.4) |
|---|---|---|---|
| A | CHEMICAL ABSTRACTS, vol. 93, no. 21, November 24, 1980, Columbus, Ohio, USA<br>SEDGWICK, EDWARD G.; MACLEOD, ROBERT A. "Energy coupling to potassium ion transport in a marine bacterium" page 353, column 1, abstract-no. 200 791r<br>& Can. J. Biochem. 1980, 58(10), 1206-14<br>-- | 1 | |
| A | CHEMICAL ABSTRACTS, vol. 92, no. 5, February 4, 1980, Columbus, Ohio, USA<br>JONES, GALEN E.; ROYLE, LESLIE G.; MURRAY, LINDSAY "Cationic composition of 22 species of bacteria grown in seawater medium" page 395, column 7, abstract-no. 37 418t<br>& Appl. Environ. Microbiol. 1979, 38(5), 800-5<br>---- | 1 | TECHNICAL FIELDS SEARCHED (Int. Cl.4) |

The present search report has been drawn up for all claims

| Place of search | Date of completion of the search | Examiner |
|---|---|---|
| VIENNA | 10-09-1987 | HAMMER |

**CATEGORY OF CITED DOCUMENTS**

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO Form 1503. 03.82